# EUROPEAN PATENT SPECIFICATION

(11) **EP 4 212 143 B1**
(45) Date of publication and mention of the grant of the patent: **26.02.2025**
(21) Application number: 22305048.5
(22) Date of filing: 18.01.2022
(51) Int. Cl.: A61J 1/20

(54) **CONNECTOR ASSEMBLY FOR MEDICAL INJECTION DEVICE**
VERBINDERANORDNUNG FÜR EINE MEDIZINISCHE INJEKTIONSVORRICHTUNG
ENSEMBLE CONNECTEUR POUR DISPOSITIF MÉDICAL D'INJECTION

(43) Date of publication of application: 19.07.2023
(73) Proprietor: Becton Dickinson France, 38800 Le Pont de Claix (FR); Becton Dickinson Holdings Pte. Ltd., Singapore 639461 (SG)
(72) Inventor: LIANG, Rongjie, Suzhou, 215021 (CN); HUANG, Longxiang, Suzhou, 215000 (CN); YONG, Zhee Min Jimmy, 425571 Singapore (SG); HUANG, Wilson, Suzhou, 215000 (CN)
(74) Representative: Regimbeau

(56) References cited:
- WO-A1-2019/219383
- WO-A1-2021/150453
- CN-A- 104 606 745
- US-A1- 2009 036 864

## Description

### FIELD OF THE INVENTION

The present invention relates to a connector assembly including an adaptor and a cap to be connected with each other for use with a medical injection device.

### BACKGROUND OF THE INVENTION

Various types of connectors for connecting two pieces of medical equipment to each other are known and available. Some known connectors are designed to be connected to a syringe and include a hollow spike for accommodating a syringe needle. The spike has a pointed tip that is used to penetrate a septum of a vial. The spike has a communication port at the tip in such a way that fluid communication is to be established between the internal volume of the vial and that of the syringe for transfer and/or reconstitution of medication to be discharged from the syringe. For example, WO 2019/219383 discloses a connector for connecting a medical injection device to a container by screwing a sleeve of the injection device into the connector at one end and connecting the container to the connector at another end.

Prior to use, the spike of such a connector often needs to be covered by a cap. For example, if the connector is used with a prefilled syringe, the communication port of the spike needs to be securely sealed during storage and transportation. Accordingly there is need for a connector assembly that can provide reliable sealing at a communication port formed in a spike.

### SUMMARY OF THE INVENTION

The present invention has been made in view of the need as described above and thus provides a connector assembly that reliably seals a communication port formed at a tip of a spike.

According to the present invention, there is provided a connector assembly as defined in claim 1 of the appended set of claims. Other advantageous embodiments are defined in the corresponding dependent claims.

The present invention also discloses a connector assembly comprising an adaptor and a cap to be connected to each other, wherein the adaptor comprises: a proximal connection portion configured to be removably connectable to a medical injection device; and a spike extending distally from the proximal connection portion and around a central axis, wherein the cap comprises a cap body defining an inner cavity extending around the central axis, the inner cavity being open at a proximal end of the cap body and is closed at a distal end of the cap body, wherein the connector assembly is configured such that the cap is removably connected to the adaptor in such a way that the spike is accommodated within the inner cavity, and wherein the cap is removably connected to the adaptor by press-fit between the spike and the inner cavity.

According to one aspect of the disclosure, the cap may further include threads on an outer circumference of the cap body, the adaptor further including a flange extending distally from the proximal connection portion and radially outward of the spike around the central axis.

According to one aspect of the disclosure, the cap may further include a stopper disposed in a helical groove delimited by the threads, the engaging protrusion being formed with a locking groove configured to receive the stopper when the cap is connected to the adaptor.

According to one aspect of the disclosure, the cap body may have an outer circumference around the central axis, the outer circumference including at least one tooth in the form of a spiral, the adaptor further including a flange extending distally from the proximal connection portion and radially outward of the spike around the central axis.

According to one aspect of the disclosure, the adaptor may further include a rim extending around the central axis at a distance distally away from the flange, the cap further including a collar extending distally from the cap body and radially outward of the cap body around the central axis, the collar being configured to rest on the rim when the cap is assembled with the adaptor.

According to one aspect of the disclosure, the collar may have a ring shape.

According to one aspect of the disclosure, the adaptor may further include at least one retention barb extending proximally from the rim and being slanted toward the central axis, the retention barb being configured to be engageable with a vial that is to be removably connected to the adaptor.

According to one aspect of the disclosure, the cap may further include at least one locking claw configured to be engageable with the retention barb.

According to one aspect of the disclosure, the adaptor may further include at least one retention barb extending distally from the flange and being configured to be engageable with a vial that is to be removably connected to the adaptor.

According to one aspect of the disclosure, the cap may further include at least one locking claw configured to be engageable with the retention barb.

According to one aspect of the disclosure, the at least one locking claw may have a forked tip.
According to one aspect of the disclosure, the adaptor may further include a flange extending distally from the proximal connection portion and radially outward of the spike around the central axis, the flange including on its inner surface at least one support rib protruding radially inwardly.

According to the connector assembly disclosed herein, the secure sealing between the adaptor spike and the cap is maintained for an extended period of time. For use, the cap can be easily removed by rotating the cap around the central axis and then pulling the cap off the adaptor. Thus, handling of the connector assembly is also facilitated.

### BRIEF DESCRIPTION OF THE FIGURES

A variety of examples of connector assemblies will be described in further detail with reference to the accompanying drawings, in which:
figure 1 is an exploded perspective view showing a connector assembly including an adaptor and a cap together with a medical injection device;
figure 2 is a perspective view showing the connector assembly of figure 1 connected to the medical injection device;
figure 3 is a side view showing the adaptor of the connector assembly of figure 1;
figure 4 is another side view showing the adaptor by rotating the view of figure 3 around a central axis by 90 degrees;
figure 5 is a side view showing the cap of the connector assembly of figure 1;
figure 6 is another side view showing the cap by rotating the view of figure 5 around the central axis by 90 degrees;
figure 7 is a longitudinal section view showing the adaptor and the cap arranged in alignment around the central axis prior to assembly;
figure 8 is a longitudinal section view showing the adaptor and the cap connected to each other;
figure 9 is another longitudinal section view showing the adaptor and the cap by rotating the view of figure 8 around the central axis by 90 degrees;
figure 10 is a longitudinal section view showing a vial and the adaptor arranged in alignment around the central axis prior to connection;
figure 11 is a longitudinal section view showing the vial and the adaptor connected to each other;
figure 12 is an enlarged, longitudinal section view showing the process of connecting the vial to the adaptor;
figure 13 is a perspective view showing a cap according to another example;
figure 14 is a side view showing a part of the adaptor of figure 12 connected with the cap;
figure 15 is a perspective view showing a cap according to another example;
figure 16 is a perspective view showing a cap according to another example;
figure 17 is a longitudinal section view showing a part of the adaptor and the cap according to another example;
figure 18 is an exploded perspective view showing a connector assembly including an adaptor and a cap according to another example;
figure 19 is a longitudinal section view showing the adaptor and the cap of figure 18 connected to each other; and
figure 20 is an exploded perspective view showing a connector assembly including an adaptor and a cap according to another example.

### DETAILED DESCRIPTION OF EMBODIMENTS OF THE INVENTION

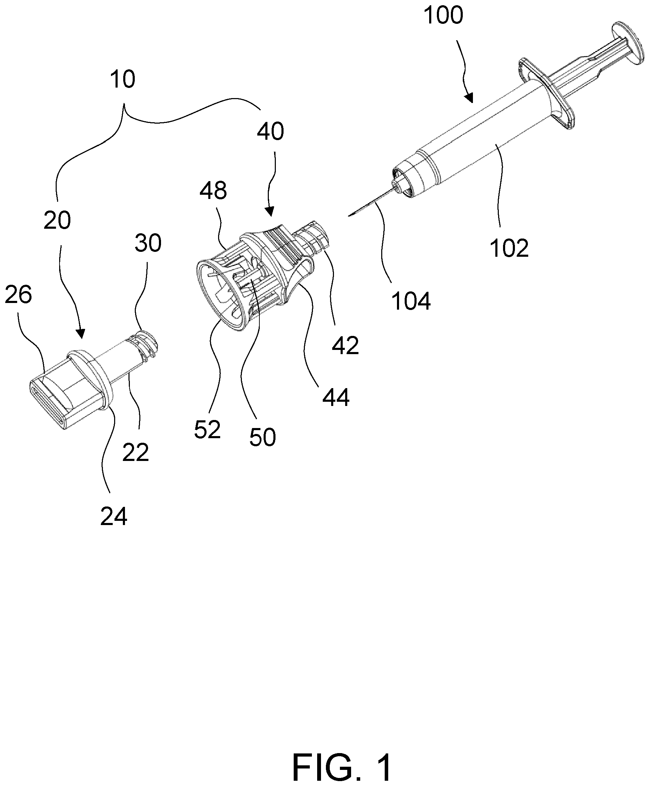

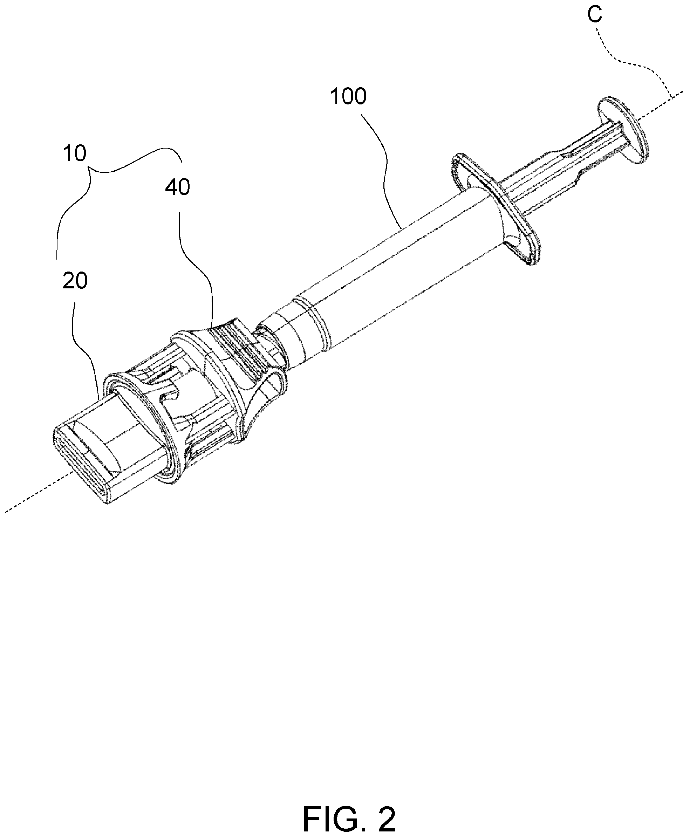

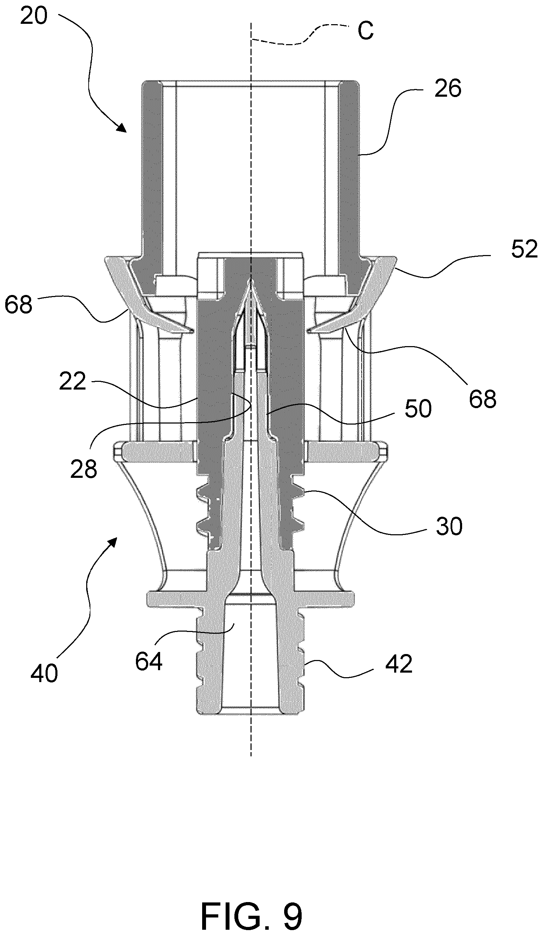

Referring to figures 1 to 9, a connector assembly 10 according to one aspect of the disclosure will be explained. Figure 1 is an exploded perspective view showing the connector assembly 10 together with a medical injection device 100. Figure 2 is a perspective view showing the connector assembly 10 connected to the injection device 100.

The connector assembly 10 is configured to be removably connected to the injection device 100. For example, the connector assembly 10 may be screwed into a barrel 102 of the injection device 100. The connector assembly 10 may be used to cover a needle 104 of the injection device 100 in order to prevent possible contamination and/or needlestick injury. The injection device 100 may be any type of injection devices available, including but not being limited to a medical syringe, in particular a prefilled syringe.

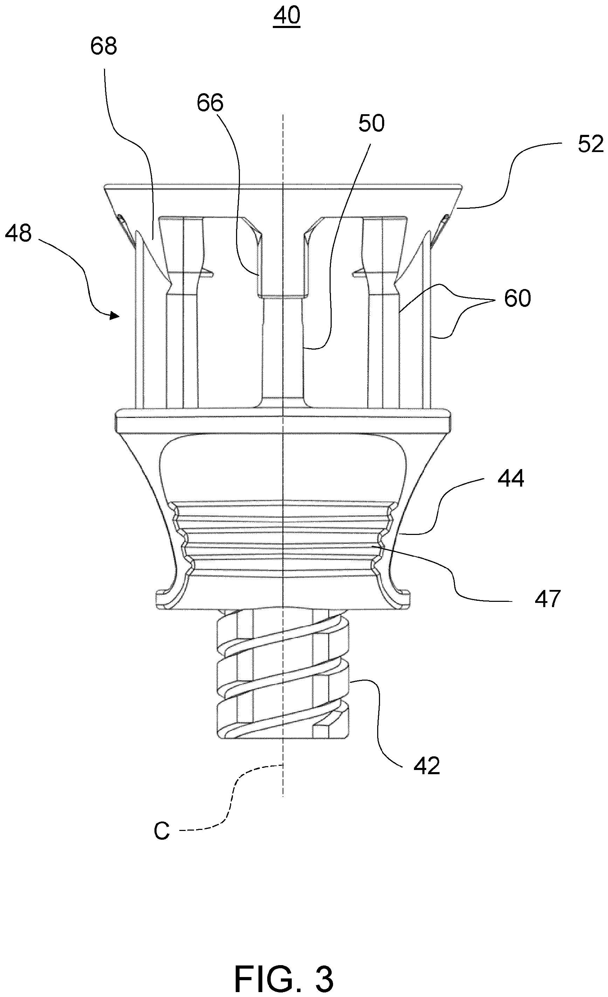

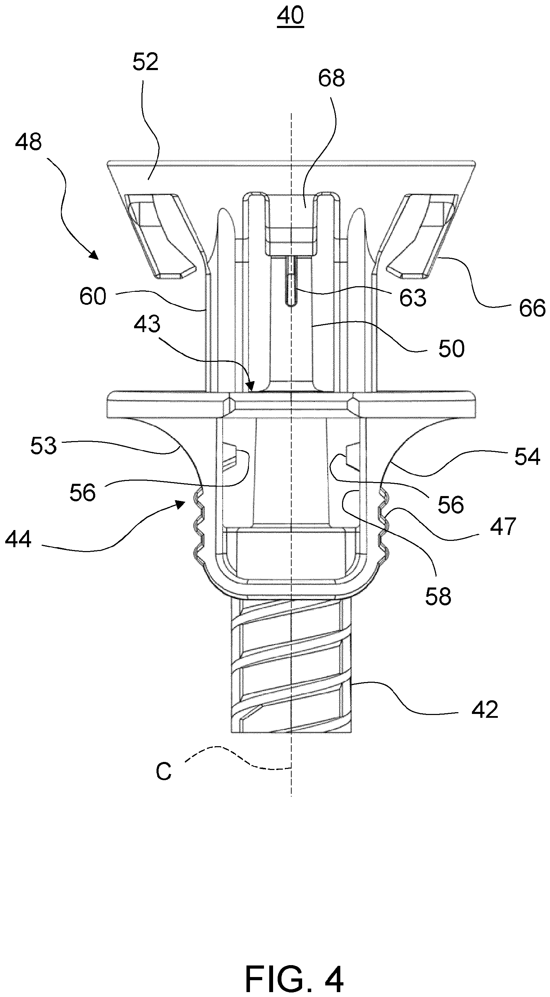

As shown in figure 2, when the connector assembly 10 and the injection device 100 are connected to each other, the connector assembly 10 and the injection device 100 are in axial alignment so as to extend around a common axis C (see figures 3 and 4, for example). The common axis C is herein referred to as a "central axis" C. Herein, a direction pointing from the central axis C to its circumference is referred to as "radially outward" and a direction opposite to the radially outward is referred to as "radially inward".

The expression "proximal" or "proximally" refers to a point or a portion closer to the end of the connector assembly 10 where the injection device 100 is to be connected. The expression "distal" or "distally" refers to a point or a portion closer to another end of the connector assembly 10.

The connector assembly 10 includes a cap 20 and an adaptor 40. The adaptor 40 serves as a luer fitting adaptor configured to connect the injection device 100 to another medical equipment. The cap 20 serves as a luer fitting cap to be connected to the adaptor 40 to provide sealing. The adaptor 40 and the cap 20 are made of a plastic material suitable for injection molding, respectively. The adaptor 40 and the cap 20 may be made of the same material or of different materials. The material of which the adaptor 40 and/or the cap 20 is/are made may include, but is not limited to, polypropylene (PP), polycarbonate (PC), acrylonitrile butadiene styrene (ABS) or acrylic.

Figures 3 and 4 show the adaptor 40 in different views rotated by 90 degrees around the central axis C. The adaptor 40 may include a proximal connection portion 42, a flange 44, a skirt 48 and a drug transfer channel such as a spike 50.

The proximal connection portion 42 is formed at a proximal end of the adaptor 40 where the adaptor 40 is to be connected to the injection device 100. The proximal connection portion 42 may be a generally tubular element extending around the central axis C and configured to be removably connected to the injection device 100. The proximal connection portion 42 may be configured to be sealingly fitted onto a tip of the barrel 102 of the injection device 100. For example, the proximal connection portion 42 may be threaded so as to be screwed onto the barrel 102 of the injection device 100, said injection device 100 comprising a corresponding thread. The thread provided on the proximal connection portion 42 is provided on the outer surface of the proximal connection portion 42 of the adaptor 40. The fitting between the adaptor 40 and the injection device 100 is preferably configured as luer-fitting.

The spike 50 extends distally from the proximal connection portion 42 around the central axis C. The spike 50 may have a generally cylindrical base and a generally conical tip extending distally from the cylindrical base. The spike 50 defines an inner channel 64 extending around the central axis C, adapted to accommodate the needle 104 of the injection device 100. The spike 50 has a communication port 63 at or near its pointed distal tip, through which fluid communication with the internal volume of the injection device 100 is made possible. The spike 50 is connected to the proximal connection portion 42.

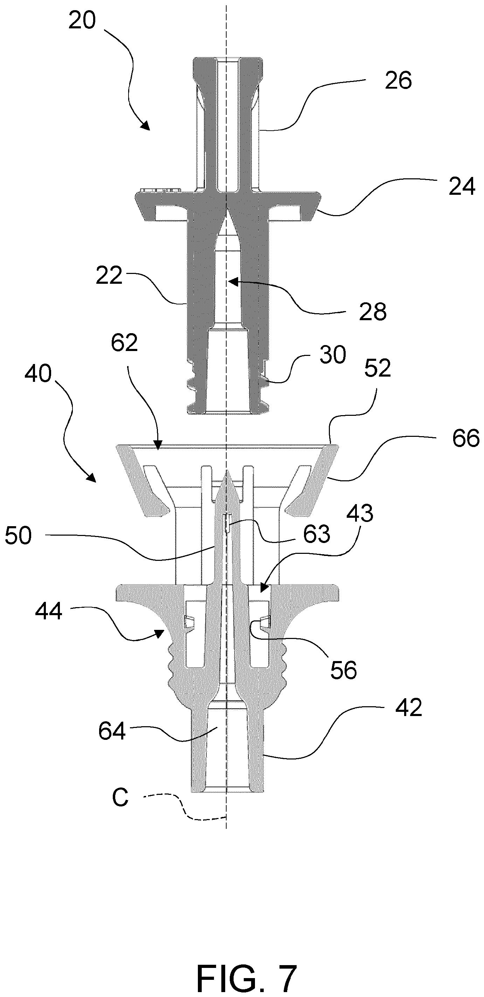

The flange 44 may extend distally from the proximal connection portion 42 and radially outward of the spike 50. The flange 44 may define a circular opening in such a way that the flange 44 and the spike 50 together define an annular gap 43 around the central axis C (see also figure 7) where the cap 20 is to be received. The flange 44 may include a pair of opposite walls 53 and 54 whose outer surfaces diverge radially outward along the central axis C. The flange 44 may be open over a part of its circumference around the central axis C, i.e. having no wall over a part of the circumference. Alternatively, the flange 44 may also form a continuous wall all around the axis C.

The flange 44 may include engaging protrusions 56 protruding radially inward from the respective inner surfaces 58 of the opposite walls 53 and 54. The engaging protrusion 56 may be formed with a locking groove (not shown). The flange 44 may include an undulating portion 47 on the outer surfaces of the opposite walls 53 and 54. The undulating portion 47 may be ergonomically designed to provide a firm grip by a user who handles the adaptor 40.

The skirt 48 may extend distally from the flange 44 and around the central axis C. At its distal end, the skirt 48 may include a rim 52 that defines a distal end of the adaptor 40. The skirt 48 may include a plurality of columns 60 connecting the flange 44 and the rim 52 to each other. Accordingly between the columns 60 through holes are provided onto the skirt 48.

The rim 52 delimits a circular opening 62 around the central axis C (see figure 7, for example). The circular opening 62 is configured to be fitted onto a corresponding portion of the cap 20 when the cap 20 is assembled with the adaptor 40; or to a corresponding portion of a vial when a vial is assembled with the adaptor 40. The rim 52 may be situated at a position distally farther from the spike 50 to protect the needle 104 within the inner channel 64 of the spike 50.

The skirt 48 may include at least one retention barb 66 extending proximally from the rim 52. The retention barb 66 is slanted with respect to the central axis C so as to be oriented radially inward. The retention barb 66 is configured to be snap-fitted onto a vial that is to be connected with a medical device via the adaptor 40, as described later in details. The skirt 48 may include two or more retention barbs 66 over its circumference around the central axis C.

The skirt 48 may also include at least one audible barb 68. Similar to the retention barb 66, the audible barb 68 extends proximally from the rim 52 and is slanted so as to be oriented radially inward. The audible barb 68 is thinner and longer than the retention barbs 66. The audible barb 68 may extend farther toward the central axis C than the retention barb 66. The audible barb 68 is configured to produce a sharp sound when the corresponding vial is fully inserted to the adaptor 40 as described later in details. The skirt 48 may include two or more audible barbs 66 over its circumference around the central axis C. The retention barbs 66 may be arranged alternately with the audible barbs 68 around the central axis C.

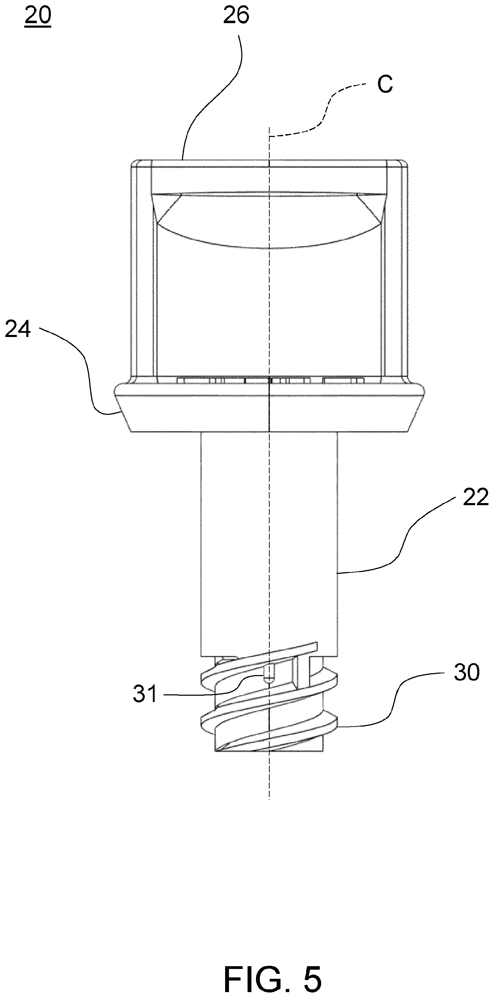

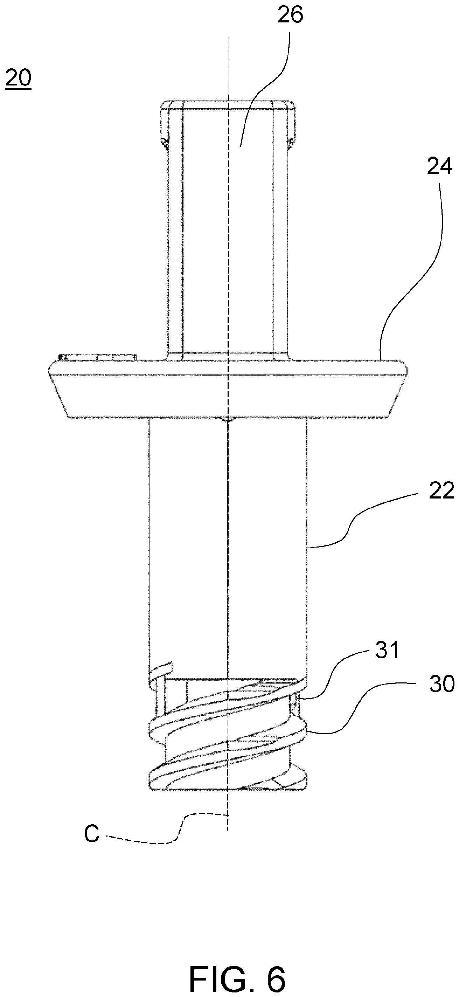

Turning to figures 5 and 6, the cap 20 will be explained. Figures 5 and 6 respectively show the cap 20 in different views rotated by 90 degrees around the central axis C. The cap 20 may include a cap body 22, a collar 24 and a tab 26.

The cap body 22 is formed with an inner cavity 28 extending around the central axis C (see figure 7). The cavity 28 opens at a proximal end of the cap body 24 and configured to accommodate the spike 50 of the adaptor 40 when the cap 20 is connected to the adaptor 40. The cavity 28 is closed at the opposite end, thereby allowing the communication port 63 to be sealed and preventing contamination of the needle 104 of the injection device.

The cap body 22 may have threads 30 at the proximal end of the cap 20. The cap body 22 may be formed with a stopper 31 in the form of a protrusion disposed in a helical groove delimited by the threads 30. The stopper 31 may be configured to be engaged with a locking groove of the adaptor 40, e.g. a small recess formed on the engaging protrusion 56, in order to prevent slipping off of the cap 20 from the connected position.

The collar 24 is configured to be engaged with the rim 52 of the adaptor 40. The collar 24 may have a generally disk shape extending around the central axis C. The collar 44 has a greater diameter than the cap body 22. The collar 24 may be delimited by a tapered circumference having a diameter that gradually decreases toward the proximal end.

The tab 26 may extend distally from the collar 24. The tab 26 may be a generally flat, rectangular parallelepiped element. The tab 26 may be ergonomically designed to allow a user to firmly hold the cap 20 by two fingers.

Hereafter the process of connecting the cap 20 with the adaptor 40 to form the connector assembly 10 will be described.

As shown in figure 7, the cap 20 and the adaptor 40 are brought in alignment around the central axis C. The cap 20 is then moved toward the adaptor 40 along the central axis C, or *vice versa*, whereby the cap body 22 is inserted into the adaptor 40 through the circular opening 62. The cap 20 further advances so that the spike 50 enters the inner cavity 28 of the cap body 22 and the cap body 22 passes through the annular gap 43 around the spike 50. Since the inner cavity 28 is designed to have a slightly smaller diameter than the outer diameter of the spike 50, the cap body 22 is moved proximally with pushing force against friction.

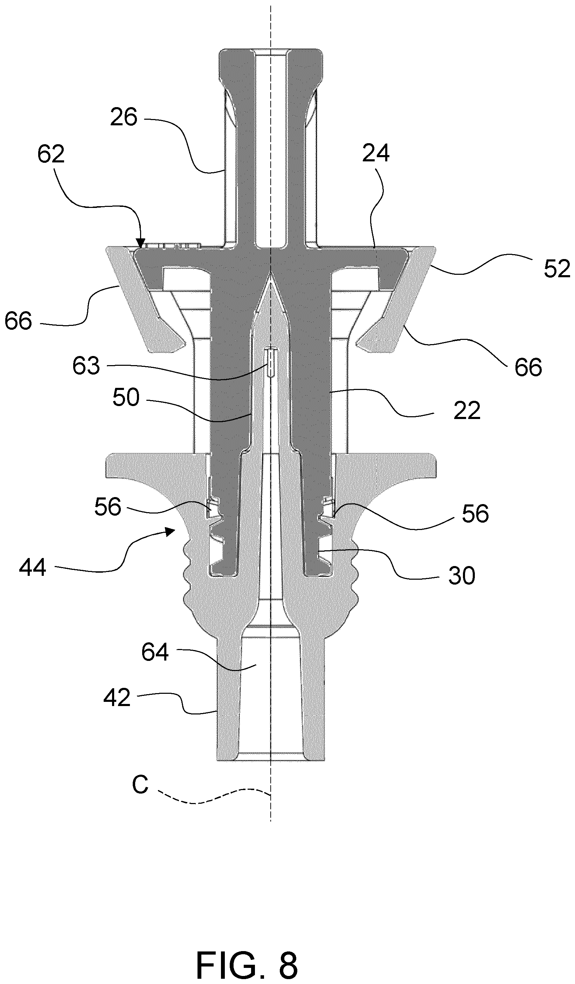

Once the proximal end of the cap 20 reaches the engaging protrusions 56 of the adaptor 40, the cap 20 is pushed farther and rotated around the central axis C in such a way that the advancement of the cap 20 is guided by engagement between the threads 30 and the engaging protrusions 56. At the end of the insertion, the stoppers 31 of the cap 20 are received by the respective locking grooves of the engaging protrusions 56, whereby the connection process of the cap 20 and the adaptor 40 is complete. The stoppers 31 and the corresponding locking grooves serve as a turn limit for preventing excessive rotation of the cap 20 and provides anti-slipping function. When the cap 20 is fully inserted into the adaptor 40, the collar 24 of the cap 20 rests on the rim 52 of the adaptor 40 (see figure 8).

When the cap 20 is taken apart from the adaptor 40, the cap 20 is rotated around the central axis C, disengaging the stopper 31 from the locking groove. By further rotating the cap 20, the engaging protrusion 56 is disengaged from the threads 30. After that, the cap 20 can be simply pulled away from the adaptor 40.

According to the connector assembly 10 as described above, the spike 50 is press-fitted into the inner cavity 28 of the cap body 22. Thus, any leakage of the liquid content of the injection device 100 through the interface between the cap 20 and the adaptor 40 is prevented. Further, in the embodiment where the cap 20 and the adaptor 40 are respectively made of a plastic material, the sealing between the two parts can be maintained over a longer period of time. In comparison, if the cap is made of an elastic material such as rubber, the sealing effect may be lost over time due to stress relaxation or deformation of the rubber cap.

Moreover, thanks to the press-fit in combination with the engagement between the threads 30 and the engaging protrusion 56, inevitable individual variation of the parts can be compensated by self-adjustment. Consequently, secure sealing between the spike 50 and the internal cavity 28 is obtained. With the optional turn limit and anti-slipping feature, excessive rotation of the cap 20 can be prevented and unintended slipping of the cap 20 relative to the adaptor 40 can be prevented.

Furthermore, while the fluid-tight sealing between the spike 50 and the inner cavity 28 is maintained, the cap 20 can be easily removed from the adaptor 40 by rotational movement around the central axis C.

With the optional open design of the flange 44 of the adaptor 40 around the central axis C, the engaging protrusion 56 can be easily produced by injection molding with simpler molding tools.

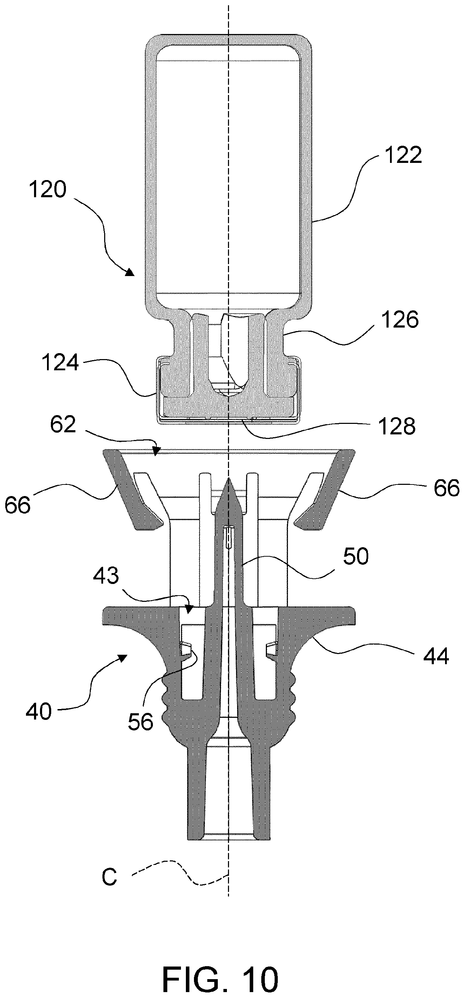

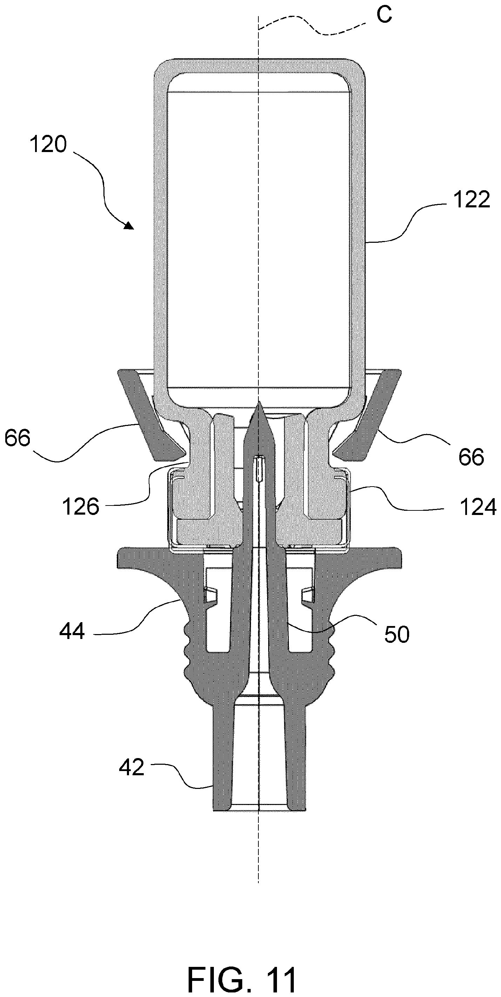

Referring to figures 10 to 12, a process of connecting the medical injection device 100 to a vial 120 via the adaptor 40 will be explained. In figures 10 and 11, the injection device 100 is omitted for the sake of better visibility.

The vial 120 may have a generally cylindrical vial body 122. The vial 120 may have a lip 124 defining an opening covered by a septum 128. A neck 126 is formed between the vial body 122 and the lip 124. The vial 120 has a smaller diameter at the neck 126 than those of the body 122 and the lip 124.

When moving the vial 120 toward the adaptor 40 or *vice versa*, the lip 124 of the vial 120 makes contact with the rim 52 of the adaptor 40. Since the rim 52 is designed to diverge radially outward, the vial 120 is easily aligned with the adaptor 40 around the central axis C.

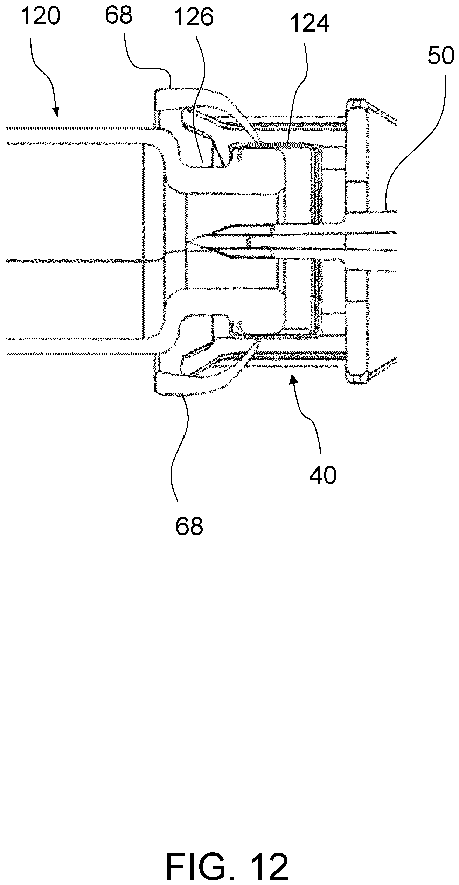

As the vial 120 advances through the circular opening 62, the lip 124 pushes and elastically deforms the audible barbs 68 substantially radially outward (see figure 12). As the vial 120 further advances, the retention barbs 66 are also subjected to elastic deformation.

When the vial 120 is fully inserted into the adaptor 40, the retention barbs 66 are disengaged from the lip 124 and rebound to be snap fitted onto the neck 126 of the vial 120. Almost simultaneously or shortly thereafter (for example, several milliseconds later), the audible barbs 68 rebound and snap fit onto the neck 126 of the vial 120. As compared to the thicker and shorter retention barbs 66 which deform slowly and to a lesser degree, the audible barbs 68 make a sudden contact with the vial neck 126 with stronger restoration force, thereby producing a sharp noise. The specific design of the audible barbs 66 can be optimized by taking into account various factors, including but not being limited to, the rigidity of materials of which the audible barbs 66 and/or the vial 120 is/are made of, the sizes of the lip 124 and the neck 126 of the vial 120.

The sharp noise resulting from the contact between the audible barbs 68 and the vial neck 126 serves as indication of full insertion of the vial 120 into the adaptor 40, i.e. the spike 50 penetrating the septum 128 of the vial 120. Thus, the user will be made aware that the vial 120 and the injection device 100 are in fluid communication and ready for transfer and/or reconstitution of the medication. In this way, the risk of any leakage between the via 120 and the adaptor 40 can be prevented.

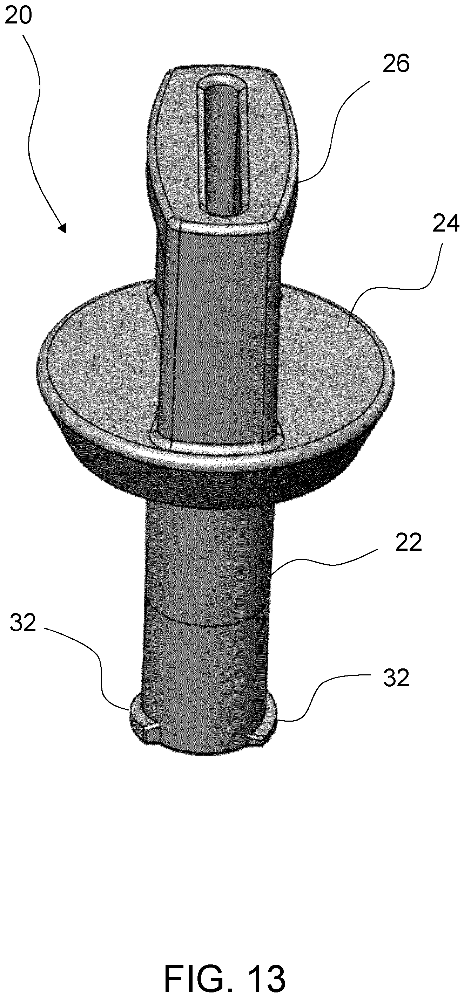

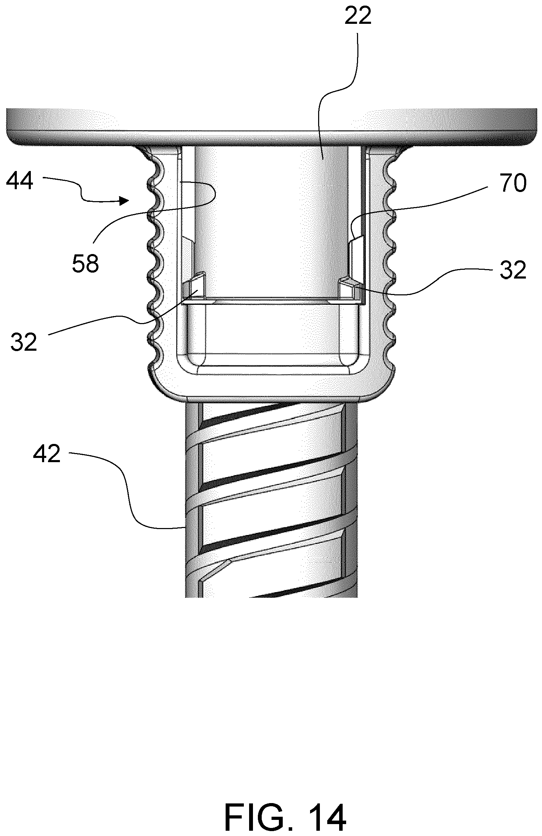

Figure 13 shows another example of the cap 20. The cap 20 in this example has the same configuration and functions as the cap 20 described above, except that the cap 20 has at least one tooth 32 at its proximal end. The tooth 32 may be generally in the form of a spiral. If more than one tooth 32 are provided, the teeth 32 may be opposite to one another with respect to the central axis C. As shown in figure 14 by way of example, the teeth 32 are configured to be engaged with corresponding ribs 70 formed on the inner surface 58 of the flange 44. The number of teeth 32 is not limited. The spike 50 and the cap body 22 are connected to each other by press-fit.

The cap 20 may have three or more teeth. The teeth 32 and/or the ribs 70 may have a ramp angle with respect to the circumference around the central axis C. With this design, the connector assembly 10 provides self-adjustment functioning in order to compensate individual variation of the parts. The teeth 32 and the ribs 70 are easily disengaged by rotational movement. Thus, the cap 20 is easily removed from the adaptor 40 in the same manner as the example described above, by rotating the cap 20 around the central axis C and subsequent pulling action along the central axis C.

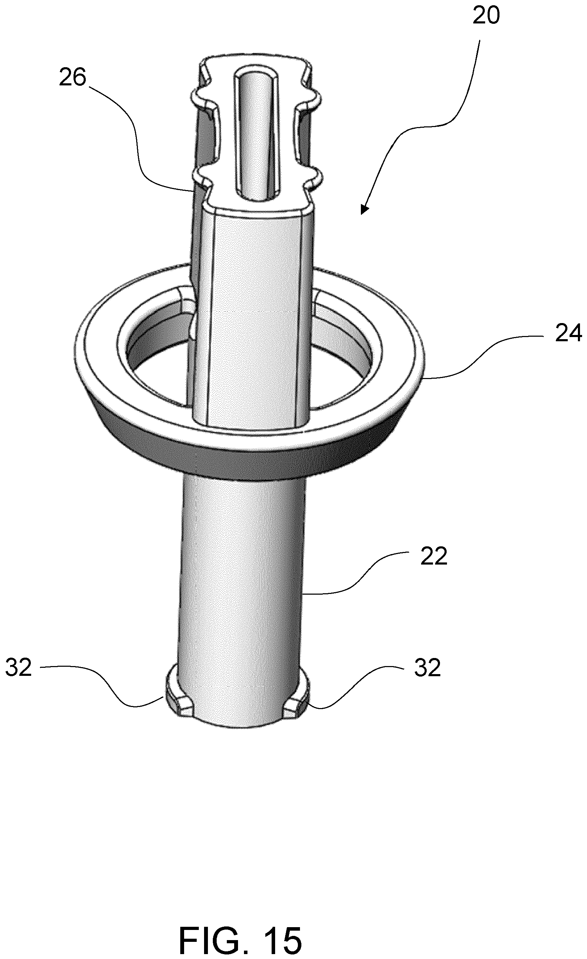

The cap 20 shown in figure 13 may be made by molding that involves a side slider. In order to simplify the molding process, the collar 24 of the cap 20 may be in the form of a ring, as illustrated in figure 15. In this example, the molded cap 20 can be released in an axial direction, i.e. parallel to the central axis C, thereby facilitating the manufacturing process.

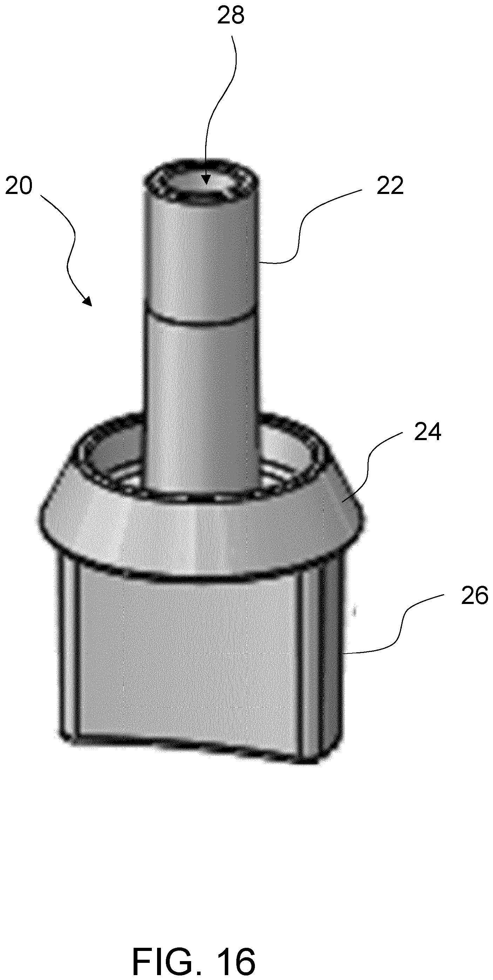

Another example of the connector assembly 10 includes a cap 20 having the same configuration and the functions as described above, except that the cap 20 includes no threads 30 nor teeth 32. As shown in figure 16, the cap 20 has a cylindrical cap body 22. The cap 20 is configured to be connectable to the adaptor 40 (not shown in figure 16) by press-fit of the spike 50 into the internal cavity 28 of the cap 20.

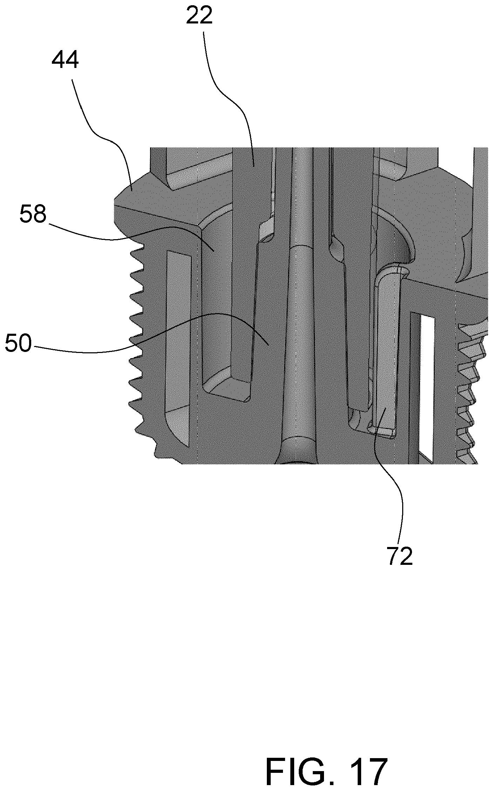

According to one example, the adaptor 40 may be formed with at least one support rib 72, as shown in figure 17. The support rib 72 protrudes radially inward from the inner surface 58 of the flange 44 and extends parallel to the central axis C. With this optional support rib 72 formed on the adaptor 40, vibration of the cap 20 can be prevented during storage and/or transportation, for example. Two or more support ribs 72 may be provided.

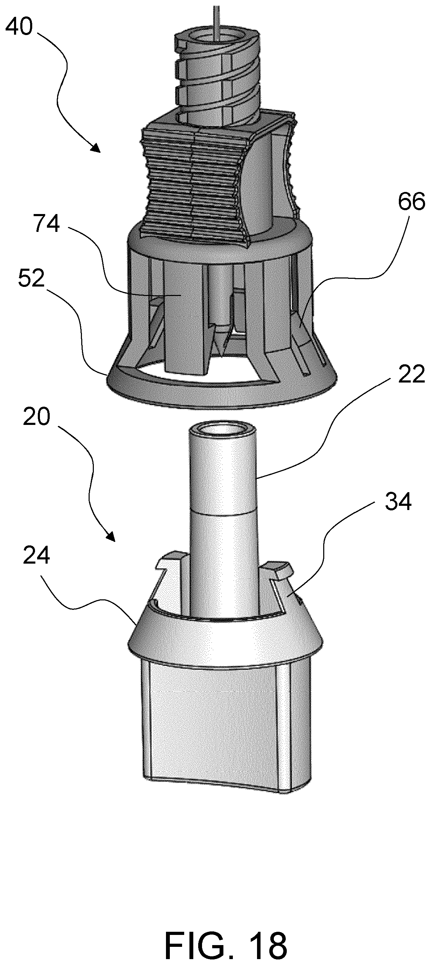

The adaptor 40 according to another example may include at least one retention barb 74 extending distally from the flange 44 and having a tip pointed radially inward, as shown in figure 18. Similar to the retention barb 66 extending proximally from the rim 52, the retention barb 74 is also configured to be snap-fitted with the vial neck 126. The retention barb 74 may be employed together with the at least one audible barb 68 as described above with reference to the previous example.

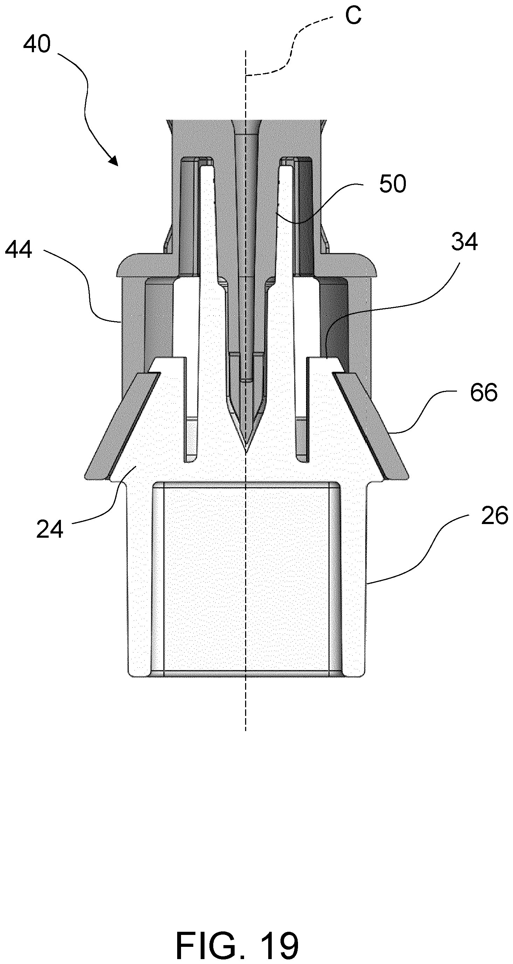

The cap 20 according to yet another example may include at least one locking claw 34 as shown in figure 18. The locking claw 34 extends proximally from the collar 24 and has a tip pointed radially outward. The locking claw 34 is configured to be engaged with the corresponding retention barbs 66 of the adaptor 40 as illustrated in figure 18. When the cap 20 is connected to the adaptor 40 as a result of axial translation along the central axis C, the retention barb 66 is to be snap fitted with the locking claw 34 (see figure 19). The engagement between the retention barb 66 and the locking claw 34 also assists in maintaining the tight connection between the cap 20 and the adaptor 40 and therefore the secure sealing between the spike 50 and the inner cavity 28. According to another example, the locking claw 34 may be configured to be engaged with the retention barb 74. It is to be noted that the rotational movement of the cap 20 around the central axis C also results in disengagement of the locking claw 34 from the retention barb 66 or from the retention barb 74. Therefore, easy removal of the cap 20 from the adaptor 40 is ensured.

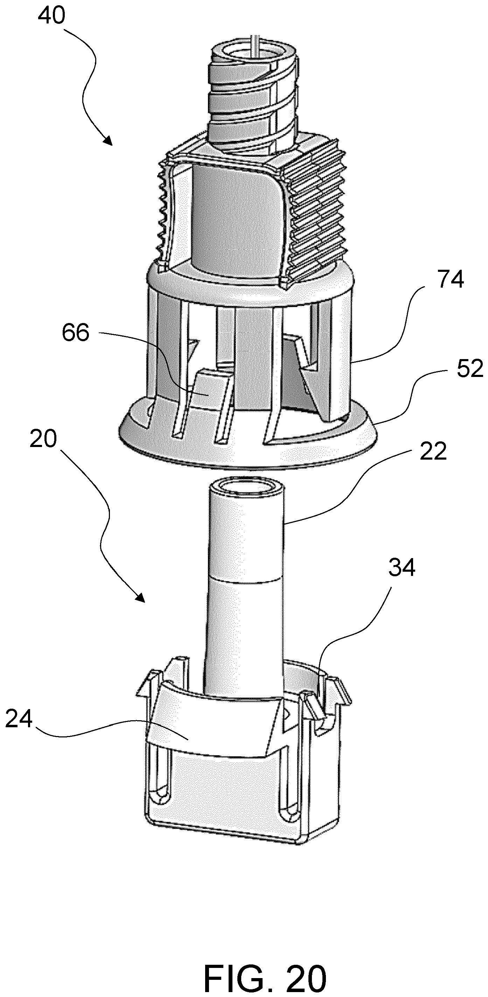

According to another example, the locking claw 34 may have a forked end as illustrated in figure 20. The locking claw 34 with the forked end may be configured to be engaged with either the retention barb 66 or the retention barb 74. With the forked end design, the locking claw 34 is relatively easily deformed, requiring low pushing force and therefore facilitating the connection process.

## Claims

1. A connector assembly (10) comprising an adaptor (40) and a cap (20) to be connected to each other,
wherein the adaptor (40) comprises:
- a proximal connection portion (42) configured to be removably connectable to a medical injection device (100); and
- a spike (50) extending distally from the proximal connection portion (42) and around a central axis (C),
wherein the cap (20) comprises a cap body (22) defining an inner cavity (28) extending around the central axis (C), the inner cavity (28) being open at a proximal end of the cap body (22) and is closed at a distal end of the cap body (22),
wherein the connector assembly (10) is configured such that the cap (20) is removably connected to the adaptor (40) in such a way that the spike (50) is accommodated within the inner cavity (28),
the assembly (10) being **characterized in that**
the adaptor and the cap are made of a plastic material suitable for injection molding and the cap (20) is removably connected to the adaptor (40) by press-fit between the spike (50) and the inner cavity (28).

2. The assembly (10) according to claim 1, wherein
the cap (20) further includes threads (30) on an outer circumference of the cap body (22) and wherein
the adaptor (40) further includes a flange (44) extending distally from the proximal connection portion (42) and radially outward of the spike (50) around the central axis (C).

3. The assembly (10) according to claim 2, wherein
the cap (20) further includes a stopper (31) disposed in a helical groove delimited by the threads (30) and the engaging protrusion (56) is formed with a locking groove configured to receive the stopper (31) when the cap (20) is connected to the adaptor (40).

4. The assembly (10) according to claim 1, wherein
the cap body (20) has an outer circumference around the central axis (C), the outer circumference including at least one tooth (32) in the form of a spiral and wherein
the adaptor (40) further includes a flange (44) extending distally from the proximal connection portion (42) and radially outward of the spike (50) around the central axis (C).

5. The assembly (10) according to any one of claims 1 to 4, wherein
the adaptor (40) further includes a rim (52) extending around the central axis (C) at a distance distally away from the flange (44), and wherein
the cap (20) further includes a collar (24) extending distally from the cap body (22) and radially outward of the cap body (22) around the central axis (C), the collar (24) being configured to rest on the rim (52) when the cap (20) is assembled with the adaptor (40).

6. The assembly (10) according to claim 5, wherein the collar (24) has a ring shape.

7. The assembly (10) according to claim 5 or 6, wherein the adaptor (40) further includes at least one retention barb (66) extending proximally from the rim (52) and being slanted toward the central axis (C), the retention barb (66) being configured to be engageable with a vial (120) that is to be removably connected to the adaptor (40).

8. The assembly (10) according to claim 7, the cap (20) further includes at least one locking claw (34) configured to be engageable with the retention barb (66).

9. The assembly (10) according to claim 5 or 6, wherein the adaptor (40) further includes at least one retention barb (74) extending distally from the flange (44) and being configured to be engageable with a vial (120) that is to be removably connected to the adaptor (40).

10. The assembly (10) according to claim 9, wherein the cap (20) further includes at least one locking claw (34) configured to be engageable with the retention barb (74).

11. The assembly (10) according to claim 8 or 10, wherein the at least one locking claw (34) has a forked tip.

12. The assembly (10) according to claim 1, wherein the adaptor (40) further includes a flange (44) extending distally from the proximal connection portion (42) and radially outward of the spike (50) around the central axis (C), the flange (44) including on its inner surface (58) at least one support rib (72) protruding radially inwardly.

## Patentansprüche

1. Verbinderbaugruppe (10), umfassend einen Adapter (40) und eine Kappe (20), die miteinander zu verbinden sind,
wobei der Adapter (40) Folgendes umfasst:
- einen proximalen Verbindungsabschnitt (42), der so konfiguriert ist, dass er entfernbar mit einer medizinischen Injektionsvorrichtung (100) verbunden werden kann; und
- einen Dorn (50), der sich distal von dem proximalen Verbindungsabschnitt (42) und um eine zentrale Achse (C) herum erstreckt,
wobei die Kappe (20) einen Kappenkörper (22) umfasst, der einen inneren Hohlraum (28) definiert, der sich um die zentrale Achse (C) erstreckt, wobei der innere Hohlraum (28) an einem proximalen Ende des Kappenkörpers (22) offen ist und an einem distalen Ende des Kappenkörpers (22) geschlossen ist,
wobei die Verbinderbaugruppe (10) so konfiguriert ist, dass die Kappe (20) mit dem Adapter (40) derart entfernbar verbunden ist, dass der Dorn (50) in dem inneren Hohlraum (28) untergebracht ist,
die Baugruppe (10) **dadurch gekennzeichnet ist, dass**
der Adapter und die Kappe aus einem Kunststoffmaterial bestehen, das zum Spritzgießen geeignet ist, und die Kappe (20) mit dem Adapter (40) durch Presspassung zwischen dem Dorn (50) und dem inneren Hohlraum (28) entfernbar verbunden ist.

2. Baugruppe (10) nach Anspruch 1, wobei
die Kappe (20) ferner ein Gewinde (30) an einem Außenumfang des Kappenkörpers (22) einschließt, wobei
der Adapter (40) ferner einen Flansch (44) einschließt, der sich distal von dem proximalen Verbindungsabschnitt (42) und radial außerhalb des Dorns (50) um die Mittelachse (C) erstreckt.

3. Baugruppe (10) nach Anspruch 2, wobei
die Kappe (20) ferner einen Stopper (31) einschließt, der in einer schraubenförmigen Nut angeordnet ist, die durch das Gewinde (30) begrenzt ist, und der Eingriffsvorsprung (56) mit einer Verriegelungsnut ausgebildet ist, die so konfiguriert ist, dass sie den Stopper (31) empfängt, wenn die Kappe (20) mit dem Adapter (40) verbunden ist.

4. Baugruppe (10) nach Anspruch 1, wobei
der Kappenkörper (20) einen Außenumfang um die Mittelachse (C) aufweist, wobei der Außenumfang mindestens einen Zahn (32) in Form einer Spirale einschließt und wobei
der Adapter (40) ferner einen Flansch (44) einschließt, der sich distal von dem proximalen Verbindungsabschnitt (42) und radial außerhalb des Dorns (50) um die Mittelachse (C) erstreckt.

5. Baugruppe (10) nach einem der Ansprüche 1 bis 4, wobei
der Adapter (40) ferner einen Rand (52) einschließt, der sich in einem Abstand distal vom Flansch (44) um die Mittelachse (C) herum erstreckt, und wobei
die Kappe (20) ferner einen Kragen (24) einschließt, der sich distal von dem Kappenkörper (22) und radial außerhalb des Kappenkörpers (22) um die Mittelachse (C) erstreckt, wobei der Kragen (24) so konfiguriert ist, dass er auf dem Rand (52) aufliegt, wenn die Kappe (20) mit dem Adapter (40) montiert ist.

6. Baugruppe (10) nach Anspruch 5, wobei der Kragen (24) eine Ringform aufweist.

7. Baugruppe (10) nach Anspruch 5 oder 6, wobei der Adapter (40) ferner mindestens einen Rückhaltewiderhaken (66) einschließt, der sich proximal von dem Rand (52) erstreckt und zur Mittelachse (C) hin geneigt ist, wobei der Rückhaltewiderhaken (66) so konfiguriert ist, dass er mit einer Phiole (120) in Eingriff gebracht werden kann, die entfernbar mit dem Adapter (40) verbunden werden soll.

8. Baugruppe (10) nach Anspruch 7, wobei die Kappe (20) ferner mindestens eine Sperrklaue (34) einschließt, die so konfiguriert ist, dass sie mit dem Rückhaltewiderhaken (66) in Eingriff gebracht werden kann.

9. Baugruppe (10) nach Anspruch 5 oder 6, wobei der Adapter (40) ferner mindestens einen Rückhaltewiderhaken (74) einschließt, der sich distal von dem Flansch (44) erstreckt und so konfiguriert ist, dass er mit einer Phiole (120) in Eingriff gebracht werden kann, die entfernbar mit dem Adapter (40) verbunden werden soll.

10. Baugruppe (10) nach Anspruch 9, wobei die Kappe (20) ferner mindestens eine Verriegelungsklaue (34) einschließt, die so konfiguriert ist, dass sie mit dem Rückhaltewiderhaken (74) in Eingriff gebracht werden kann.

11. Baugruppe (10) nach Anspruch 8 oder 10, wobei die mindestens eine Sperrklaue (34) eine gegabelte Spitze aufweist.

12. Baugruppe (10) nach Anspruch 1, wobei der Adapter (40) ferner einen Flansch (44) einschließt, der sich distal von dem proximalen Verbindungsabschnitt (42) und radial außerhalb des Dorns (50) um die zentrale Achse (C) erstreckt, wobei der Flansch (44) auf seiner inneren Oberfläche (58) mindestens eine Stützrippe (72) aufweist, die radial nach innen vorsteht.

## Revendications

1. Ensemble de connecteurs (10) comprenant un adaptateur (40) et un capuchon (20) à connecter l'un à l'autre,
dans lequel l'adaptateur (40) comprend
- une portion de connexion proximale (42) configurée pour être connectée de manière amovible à un dispositif d'injection médicale (100) ; et
- une pointe (50) s'étendant distalement à partir de la portion de connexion proximale (42) et autour d'un axe central (C),
dans lequel le capuchon (20) comprend un corps de capuchon (22) définissant une cavité intérieure (28) s'étendant autour de l'axe central (C), la cavité intérieure (28) étant ouverte à une extrémité proximale du corps de capuchon (22) et fermée à une extrémité distale du corps de capuchon (22),
dans lequel l'ensemble de connecteurs (10) est configuré de manière à ce que le capuchon (20) soit connecté de manière amovible à l'adaptateur (40) de telle sorte que la pointe (50) soit logée dans la cavité intérieure (28),
l'ensemble (10) étant **caractérisé en ce que** l'adaptateur et le capuchon sont fabriqués dans une matière plastique adaptée au moulage par injection et que le capuchon (20) est relié de manière amovible à l'adaptateur (40) par emmanchement entre la pointe (50) et la cavité intérieure (28).

2. Ensemble (10) selon la revendication 1, dans lequel
le capuchon (20) comprend en outre des filets (30) sur une circonférence extérieure du corps de capuchon (22) et dans lequel
l'adaptateur (40) comprend en outre une bride (44) s'étendant distalement à partir de la portion de connexion proximale (42) et radialement vers l'extérieur de la pointe (50) autour de l'axe central (C).

3. Ensemble (10) selon la revendication 2, dans lequel
le capuchon (20) comprend en outre un bouchon (31) disposé dans une rainure hélicoïdale délimitée par les filets (30) et la saillie d'engagement (56) est formée d'une rainure de verrouillage configurée pour recevoir le bouchon (31) lorsque le capuchon (20) est connecté à l'adaptateur (40).

4. Ensemble (10) selon la revendication 1, dans lequel
le corps du capuchon (20) a une circonférence extérieure autour de l'axe central (C), la circonférence extérieure comprenant au moins une dent (32) en forme de spirale et dans lequel
l'adaptateur (40) comprend en outre une bride (44) s'étendant distalement à partir de la portion de connexion proximale (42) et radialement vers l'extérieur de la pointe (50) autour de l'axe central (C).

5. Ensemble (10) selon l'une quelconque des revendications 1 à 4, dans lequel
l'adaptateur (40) comprend en outre un bord (52) s'étendant autour de l'axe central (C) à une distance distale de la bride (44), et dans lequel
le capuchon (20) comprend en outre une collerette (24) s'étendant distalement à partir du corps de capuchon (22) et radialement vers l'extérieur du corps de capuchon (22) autour de l'axe central (C), la collerette (24) étant configurée pour reposer sur le rebord (52) lorsque le capuchon (20) est assemblé avec l'adaptateur (40).

6. Ensemble (10) selon la revendication 5, dans lequel la collerette (24) a une forme d'anneau.

7. Ensemble (10) selon la revendication 5 ou 6, dans lequel l'adaptateur (40) comprend en outre au moins une barrette de rétention (66) s'étendant proximalement à partir du bord (52) et étant inclinée vers l'axe central (C), la barrette de rétention (66) étant configurée pour être engagée avec un flacon (120) destiné à être connecté de manière amovible à l'adaptateur (40).

8. Ensemble (10) selon la revendication 7, dans lequel le capuchon (20) comprend en outre au moins une griffe de verrouillage (34) configurée pour être en prise avec la barrette de rétention (66).

9. Ensemble (10) selon la revendication 5 ou 6, dans lequel l'adaptateur (40) comprend en outre au moins une barrette de rétention (74) s'étendant distalement à partir de la bride (44) et configurée pour être engagée avec un flacon (120) destiné à être connecté de manière amovible à l'adaptateur (40).

10. Ensemble (10) selon la revendication 9, dans lequel le capuchon (20) comprend en outre au moins une griffe de verrouillage (34) configurée pour être en prise avec la barrette de rétention (74).

11. Ensemble (10) selon la revendication 8 ou 10, dans lequel l'au moins une barrette de verrouillage (34) a une extrémité fourchue.

12. Ensemble (10) selon la revendication 1, dans lequel l'adaptateur (40) comprend en outre une bride (44) s'étendant distalement à partir de la portion de connexion proximale (42) et radialement vers l'extérieur de la pointe (50) autour de l'axe central (C), la bride (44) comprenant sur sa surface intérieure (58) au moins une nervure de support (72) faisant saillie radialement vers l'intérieur.
